(19) Europäisches Patentamt
European Patent Office
Office européen des brevets

(11) Veröffentlichungsnummer: **0 071 791**
**B1**

(12) # EUROPÄISCHE PATENTSCHRIFT

(45) Veröffentlichungstag der Patentschrift: **08.05.85**

(51) Int. Cl.⁴: **C 07 C 149/40**, C 07 C 148/00

(21) Anmeldenummer: **82106334.4**

(22) Anmeldetag: **15.07.82**

(54) **Neue Trichlormethylthiobenzoylhalogenide und Verfahren zur Herstellung von m- oder p-Trichlormethylthiobenzoylhalogeniden.**

(30) Priorität: **01.08.81 DE 3130437**

(43) Veröffentlichungstag der Anmeldung: **16.02.83 Patentblatt 83/7**

(45) Bekanntmachung des Hinweises auf die Patenterteilung: **08.05.85 Patentblatt 85/19**

(84) Benannte Vertragsstaaten: **BE CH DE FR GB LI**

(56) Entgegenhaltungen:
**DE - A - 2 917 618**
**SU - A - 163 610**

***Chemical Abstracts Band 55, Nr. 21 16. Oktober 1961 Columbus, Ohio, USA L.M. YAGUPOL'SKII et al. "p-Trichloro-methylthio- and p-trichloromethoxybenzoic acids and their derivatives" Spalte 21029a***

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Hamprecht, Gerhard, Dr., Rote-Turm-Strasse 28, D-6940 Weinheim (DE)**
Erfinder: **Reissenweber, Gernot, Dr., Pfarrer-Friedrich-Strasse 41, D-6700 Ludwigshafen (DE)**

**EP 0 071 791 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft die neuen Trichlormethylthiobenzoylhalogenide und ein Verfahren zur Herstellung von m- oder p-Trichlormethylthiobenzoylhalogeniden durch Umsetzung von m- oder p-Methylthiobenzoylhalogeniden mit Chlorgas in Abwesenheit von zusätzlichen Lösungsmitteln bei einer Temperatur von 25 bis 140°C.

Es ist bekannt, Dichlormethyl-phenylsulfid durch Chlorierung mit elementarem Chlor in Tetrachlorkohlenstoff in Trichlormethylphenylsulfid zu überführen (Ann. 581, S. 137—139 (1953)). Werden jedoch substituierte Arylsulfide eingesetzt, so treten Nebenreaktionen ein; in vielen Fällen kommt es zu einer Spaltung der Schwefel-Kohlenstoffbildung. So liefert das 3-Nitrotolyl-4-methylsulfid bei der Chlorierung in gekühltem Tetrachlorkohlenstoff unter Abspaltung des Methylrestes 3-Nitrotolyl-4-schwefelchlorid (Ann. 406, S. 127—133 (1941)). Häufig geht der Kernsubstitution eine Halogenaddition an den Schwefel voraus. So wandelt sich Phenylmethylsulfiddibromid in Tetrachlorkohlenstofflösung bei Raumtemperatur langsam, bei 87 bis 88°C rasch in p-Bromphenyl-methylsulfid um (R. 30, 407—417 (1911)). In gleicher Weise bildet sich beim Einleiten von Chlor in die gekühlte benzolische Lösung von Diphenylsulfid das Diphenylsulfiddichlorid, das schon durch den Wasserdampf der Luft unter Bildung von Diphenylsulfoxid zersetzt wird (Ann. 381, 337—343 (1911)). Auch bei Ausschluß von Wasser verändert sich das Dichlorid innerhalb kurzer Zeit, wobei das Chlor vom Schwefel in den Kern abwandert.

Unter speziellen Bedingungen konnten Yagupolskij et al. p-Trichlormethylthiobenzoylchlorid durch Chlorieren von p-Methylthiobenzoylchlorid in Chloroform-Lösung bei 10°C und unter Bestrahlung in schlechter Raum-Zeit-Ausbeute gewinnen (Ukrain. Khim. Zhur. 27, 77—79 (1961)); (CA. 55, 21 029 a). Das hierbei anfallende Rohprodukt muß noch durch Kochen mit Aktivkohle in Petrolether von schwerlöslichen Verunreinigungen getrennt werden. Bei höheren Temperaturen (40 bis 80°C) konnten Senning et al. m-Methylthiobenzoesäure in Chloroform-Lösung in 67% Ausbeute in rohe m-Trichlormethylthiobenzoesäure überführen (Acta Chem. Scand. 14, 2200—2235 (1960)). Auch hier ist der Reaktionsverlauf nicht einheitlich; das Produkt muß noch im Soxhletapparat mit Petroether extrahiert und mehrfach aus Chloroform umkristallisiert werden.

Die für die bisher bekannten Chlorierungsverfahren erforderlichen Lösungsmittel Chloroform oder Tetrachlorkohlenstoff sind jedoch sehr toxische Lösungsmittel, deren Einsatz aus der heutigen Sicht der Arbeitssicherheit zu vermeiden ist (Merkblätter Gefährliche Arbeitsstoffe, Verlag Moderne Industrie, W. Dummer & Co., München, 1977). Tetrachlorkohlenstoff wirkt ferner auch krebserzeugend (Roth. Daunderer-Giftliste, 8. Erg. Lfg. 12/80; 1979 ecomed Verlagsgesellschaft, Landsberg).

Die Patentschrift SU-A-163 610 beschreibt in drei Beispielen die Chlorierung von Phenylmethylsulfid, p-Chlorphenyl- und p-Nitrophenyl-methylsulfid, wobei als Reaktionstemperatur 70 bis 100°C angegeben wird. Dieses Verfahren liefert nur dann befriedigende Ausbeuten, wenn unsubstituierte Phenylmethylsulfid oder mit Substituenten 1. Ordnung versehene Analoge eingesetzt werden (Beispiel 1 und 2). Im Falle von Substituenten 2. Ordnung fallen Ausbeute und Reinheit stärker ab. So beträgt die Rohausbeute im Falle des p-Nitrophenyltrichlormethylsulfids 85% (Beispiel 3); das Produkt muß jedoch zuerst einmal aus Aceton umkristallisiert werden, bis es einen Schmelzpunkt von 91 bis 92°C aufweist (reines p-Nitrophenyl-trichlormethylsulfid 94°C) (Ber. 43, 3444 (1910); J. Gen. Chem. UdSSR, 22, 2273 (1952); CA. 72, 66 651).

Die DE-A-2 917 618 beschreibt ein Verfahren zur Herstellung von 3-Chlor-4-trichlormethylthionitrobenzol, bei dem das Phenylmethylsulfid durch die schon bisher verwendeten Substituenten Chlor und Nitro substituiert ist. Die RZA beträgt 0,52 kg/l/Tag (Beispiel 1).

Alle vorgenannten Verfahren sind daher in bezug auf Ausbeute und Reinheit des Endstoffs, Einheitlichkeit der Reaktion sowie sichere Handhabung unbefriedigend.

Es wurde nun gefunden, daß man m- oder p-Trichlormethylthiobenzoylhalogenide der Formel

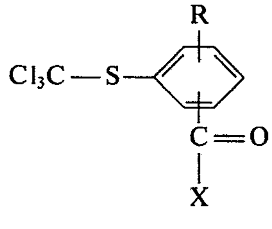

(I)

worin X ein Halogenatom bedeutet, R ein Wasserstoffatom, ein Halogenatom oder einen aliphatischen Rest bezeichnet, vorteilhaft erhält, wenn man m- oder p-Methylthiobenzoylhalogenide der Formel

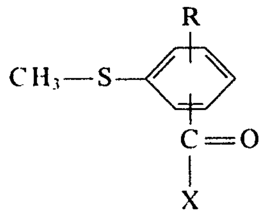

(II)

worin R und X die vorgenannte Bedeutung besitzen, mit Chlorgas in Abwesenheit von zusätzlichen Lösungsmitteln bei einer Temperatur von 25 bis 140°C umsetzt.

Weiterhin wurde gefunden, daß man das Verfahren nach Anspruch 1 vorteilhaft durchführt, wenn man die Reaktionstemperatur zu Beginn der Chlorierung bei 25 bis 45°C und dann nach kontinuierlicher Steigerung gegen Ende der Chlorierung bei 50 bis 140°C hält.

Weiterhin wurden die neuen Stoffe 3-Trichlormethylthiobenzoylchlorid und 4-Chlor-3-trichlormethylthiobenzoylchlorid gefunden.

Die Umsetzung kann für den Fall der Verwendung von p-Methylthiobenzoylchlorid durch die folgenden Formeln beschrieben werden:

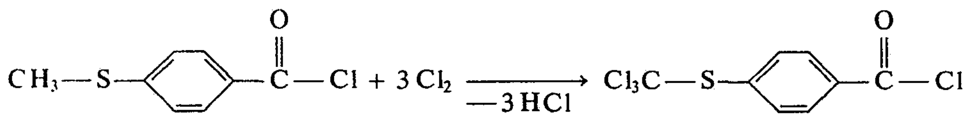


Im Vergleich zu den bekannten Verfahren liefert das Verfahren nach der Erfindung auf einfacherem und wirtschaftlicherem Wege Trichlormethylthiobenzoylhalogenide in besserer Ausbeute und Reinheit. Aufwendige bzw. toxische Lösungsmittel werden nicht verwendet. Im Hinblick auf den Stand der Technik sind alle diese vorteilhaften Eigenschaften überraschend.

Im Hinblick auf SU-A-163 610 lassen sich nach dem erfindungsgemäßen Verfahren unerwartet bekannte und neue trichlormethylthiosubstituierte Benzoylhalogenide trotz der Anwesenheit eines stark elektronenziehenden Substituenten 2. Ordnung (—COX) in fast quantitativer Ausbeute herstellen. Auch hinsichtlich der Raum-Zeit-Ausbeute (RZA) unterscheidet sich das erfindungsgemäße Verfahren von dem Verfahren dieser Patentschrift. Beträgt die Raum-Zeit-Ausbeute im Falle des desaktivierend substituierten p-Nitrophenyl-methylsulfids 6,65 kg/l/Tag, so liegt sie nach dem erfindungsgemäßen Verfahren bei ebenfalls desaktivierender Substitution überraschend um ein Mehrfaches höher.

Im Hinblick auf C. A. 55, 21 029 und Acta Chem. Scand. 14, 2230—2235 (1960), nach denen die sehr viel empfindlicheren carbonylsubstituierten Phenyltrichlormethylsulfide nur in schlechten Raum-Zeit-Ausbeuten und schlechter Reinheit zugänglich sind, ist es überraschend, daß das erfindunsgemäße Verfahren in Abwesenheit zusätzlicher Lösungsmittel und insbesondere auch bei der besonderen Temperaturführung ein wesentlich besseres Ergebnis in bezug auf Ausbeute und Reinheit liefert.

Bevorzugte Ausgangsstoffe II und dementsprechend bevorzugte Endstoffe I sind solche, in deren Formeln X ein Bromatom, Fluoratom oder Chloratom bedeutet, R für ein Wasserstoffatom, ein Bromatom oder Chloratom oder einen Alkylrest mit 1 bis 4 Kohlenstoffatomen bezeichnet. Der Methylthiorest steht in m- oder p-Stellung zur Carboxylgruppe.

So sind beispielsweise folgende Ausgangsstoffe II geeignet: m- oder p-Methylthiobenzoylchlorid; in o-, m- oder p-Stellung zur Halogencarbonylgruppe am Benzolkern durch Chloratome, Bromatome, Methyl-, Ethyl-, Propyl-, Isopropyl-, Butyl-, Isobutyl-, sek.-Butyl-, tert.-Butyl-gruppe substituierte m- oder p-Methylthiobenzoylbromide.

Ausgangsstoff II kann mit Chlor in stöchiometrischer Menge oder vorzugsweise im Überschuß, vorteilhaft mit 3,1 bis 11, insbesondere 3,3 bis 8 Mol $Cl_2$ je Mol Ausgangsstoff II, umgesetzt werden. Die Umsetzung wird bei einer Temperatur von 25 bis 140°C, vorteilhaft von 25 bis 100°C, drucklos oder unter Druck, kontinuierlich oder diskontinuierlich durchgeführt.

Chloriert man bei 1 bar, so werden zweckmäßig 3,3 bis 8 Mol Chlorgas, bezogen auf 1 Mol Ausgangsstoff II, eingesetzt, was einem Chlorumsatz von 91 bis 37% entspricht. Durch geeignete apparative Maßnahmen, z. B. durch Anwendung von mäßigem Überdruck, zweckmäßig 1 bis 10 bar, oder durch Verwendung einer Blasensäule, läßt sich der Chlorumsatz erhöhen. Vorteilhaft läßt man das Chlorgas möglichst lange mit der organischen Phase in Berührung kommen, indem diese beispielsweise stark gerührt wird oder das Chlorgas eine dicke Schicht der organischen Phase durchdringen muß.

Die Reaktionszeit beträgt im allgemeinen etwa ¼ bis 10 Stunden.

In einer bevorzugten Ausführungsform des Verfahrens geht man so vor, daß man innerhalb von ¼ bis 10 Stunden, vorzugsweise ½ bis 3 Stunden, die erforderliche Menge Chlorgas unter intensivem Rühren in den flüssigen Ausgangsstoff II einleitet, wobei man zunächst bei einer Temperatur von etwa 25 bis 45°C beginnt und diese dann während der weiteren Chlorierung kontinuierlich steigert, so daß gegen Ende die Umsetzung bei 50 und 140°C, vorzugsweise 70 bis 100°C, durchgeführt wird. Vorteilhaft ist eine Steigerung der Temperatur während der Reaktion von 1 bis 10°C pro Minute, wobei man bei der Anfangstemperatur, z. B. von 25°C, zweckmäßig 2 bis 5 Minuten verweilt und dann erst die Temperatur

steigert. Ebenfalls kann man zweckmäßig am Ende die Reaktion noch 15 Minuten bei der Endtemperatur, z. B. 140°C, vorzugsweise 100°C, halten. Die Reaktion ist stark exotherm und muß gekühlt werden; mit dem Abflauen der Reaktion entfernt man das Kältebad und kann gegebenenfalls noch kurz nacherhitzen.

Gegebenenfalls kann man die Reaktion durch Zugabe eines Katalysators beschleunigen; als solcher eignet sich Phosphorpentachlorid in zweckmäßig einer Menge von 0,5 bis 7 Mol-%, bezogen auf den Ausgangsstoff II. In diesem Fall legt man den Ausgangsstoff II zusammen mit dem Katalysator vor und beginnt dann mit der Chlorierung. Statt des Phosphorpentachlorids kann man auch eine dieses unter den Reaktionsbedingungen bildende Ausgangskomponente, z. B. Phosphortrichlorid oder gelben Phosphor, zugeben und dann mit der Chlorierung beginnen.

Die Aufarbeitung und Isolierung der Endstoffe kann in üblicher Weise erfolgen. Beispielsweise kann man aus der heißen organischen Phase mittels eines Inertgases Reste an Chlorwasserstoff, Chlor oder Katalysator austragen; dabei hinterbleibt in praktisch quantitativer Ausbeute ein bereits sehr reines Rohprodukt. Dieses kann zwar weiter gereinigt werden, beispielsweise durch Destillation, ist aber bereits genügend rein, um direkt für weitere Umsetzungen eingesetzt werden zu können.

Die so herstellbaren Trichlormethylthiobenzoylhalogenide, insbesondere die Chloride, sind technisch wichtige Zwischenprodukte für die Herstellung selektiv wirkender Herbizide, z. B. von 4 H-3,1-Benzoxazinderivaten (DE-PS 2 914 915). Hierbei werden die erfindungsgemäß zugänglichen Trichlormethylthiobenzoylchloride mit Fluorwasserstoff in entsprechende Trifluormethylthiobenzoylfluoride umgewandelt und dann mit Anthranilsäure nach dem Verfahren der deutschen Patentanmeldung 2 914 915 zu 4 H-3,1-Benzoxazinen umgesetzt. 2-(Trifluormethylthio)-3,1-benzoxazin(4)-on wurde früher auf Basis m-Trifluormethylmercaptoanilin über eine Sandmeyer-Reaktion, Verseifung zur Säure und deren Überführung in das 3-Trifluormethylthiobenzoylchlorid hergestellt. Aus toxikologischen Gründen kann 3-Trifluormethylmercaptoanilin nicht im Betriebsmaßstab hergestellt werden. Voraussetzung für die sichere Herstellung des Wirkstoffes ist daher die durch das erfindungsgemäße Verfahren geschaffene Möglichkeit, reines 3-Trichlormethylthiobenzoylchlorid herzustellen.

Die in den folgenden Beispielen genannten Teile sind Gewichtsteile. Sie verhalten sich zu den Volumenteilen wie Kilogramm zu Liter.

## Beispiel 1

### 3-Trichlormethylthiobenzoylchlorid

a) In eine Mischung von 100 Teilen 3-Methylthiobenzoylchlorid und 2 Teilen Phosphorpentachlorid wird unter starkem Rühren, beginnend bei 25°C und unter äußerer Kühlung, Chlorgas eingeleitet, wobei die Temperatur in 4 Minuten auf 45°C ansteigt. Bei Verstärkung des Chlorgasstromes und unter weiterer Kühlung steigt die Innentemperatur auf 80°C. Insgesamt werden während 35 Minuten 175 Teile Chlorgas eingeleitet, hierbei muß während der ersten 25 Minuten gekühlt werden. Bei der anschließenden Destillation werden bei 102 bis 108°C/0,4 mbar 151 Teile (97% der Theorie) 3-Trichlormethylthiobenzoylchlorid mit $n_D^{25} = 1{,}6070$ erhalten.

b) Bei Verwendung von 6,6 Teilen Phosphorpentachlorid als Katalysator und gleicher Temperaturführung wie in a), jedoch Chlorierung am Schluß bei 110°C während 8 Minuten (Gesamtreaktionszeit 28 Minuten), wird die gleiche Ausbeute und Reinheit an 3-Trichlormethylthiobenzoylchlorid erhalten.

c) Unter starkem Rühren werden insgesamt 250 Teile Chlorgas bei 30 bis 51°C während 65 Minuten in 100 Teile 3-Methylthiobenzoylchlorid eingeleitet. Bei der Destillation werden 144,4 Teile (93%) 3-Trichlormethylthiobenzoylchlorid vom Kp 102 bis 108°C/0,4 mbar erhalten.

## Beispiel 2

### 4-Chlor-3-trichlormethylthiobenzoylchlorid

a) 182 Teile Dimethylsulfat werden bei 25 bis 30°C unter Rühren zu einer Lösung von 92 Teilen 4-Chlor-3-thiobenzoesäure in 1000 Volumenteilen 3 n-Natronlauge gegeben und 10 Stunden bei Raumtemperatur (20°C) nachgerührt. Das Reaktionsgemisch wird angesäuert, abgesaugt und getrocknet, wobei die 4-Chlor-3-methylthiobenzoesäure mit Fp 210 bis 215°C anfällt.

b) 98 Teile der so erhaltenen Säure werden in 500 Teilen 1,2-Dichlorethan suspendiert, mit einem Tropfen Pyridin als Katalysator und unter Rühren mit 70 Teilen Thionylchlorid versetzt. Nach 2 Stunden Rühren unter Rückfluß wird das Reaktionsgemisch eingeengt, wobei das 4-Chlor-3-methylthiobenzoylchlorid vom Kp 108 bis 112°C/0,5 mbar, Fp 37 bis 39°C, erhalten wird.

c) In eine Schmelze von 75 Teilen 4-Chlor-3-methylthiobenzoylchlorid und 1,5 Teilen Phosphortrichlorid wird, beginnend bei 45°C, unter Rühren Chlorgas eingeleitet, wobei bei nun zugeschalteter Außenkühlung die Innentemperatur innerhalb von 5 Minuten auf 70°C ansteigt. Während weiterer

45 Minuten wird dann in einem Temperaturbereich von 68 bis 74°C und zum Schluß unter Erhitzen während 10 Minuten bei 90°C insgesamt eine Menge von 192 Teilen Chlorgas eingeführt. Bei der anschließenden Destillation werden 98,9 Teile (90% der Theorie) 4-Chlor-3-trichlormethylthiobenzoylchlorid vom Kp 127 bis 134°C/0,5 mbar erhalten.

Beispiel 3

4-Trichlormethylthiobenzoylchlorid

Beginnend bei 25°C werden insgesamt 114 Teile Chlor unter intensivem Rühren in 50 Teile 4-Methylthiobenzoylchlorid eingeleitet. Die Reaktion ist exotherm und wird durch Kühlung während der ersten 10 Minuten zunächst bei 25 bis 60°C durchgeführt. Während 20 Minuten wird Chlor eingeleitet, wobei im Reaktionsgefäß die Innentemperatur innerhalb von 10 Minuten von 25 bis 60°C und 10 Minuten von 60 auf 90°C steigt, unter Beheizen wird nun noch 15 Minuten bei 100°C chloriert. Bei der Destillation bei 115 bis 120°C/0,4 mbar werden 73,1 Teile (94% der Theorie) praktisch farbloses 4-Trichlormethylthiobenzoylchlorid mit Fp 48 bis 51°C erhalten.

**Patentansprüche**

1. Verfahren zur Herstellung von m- oder p-Trichlormethylthiobenzoylhalogeniden der Formel

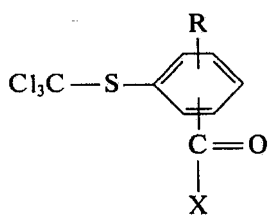

(I)

worin X ein Halogenatom bedeutet, R ein Wasserstoffatom, ein Halogenatom oder einen aliphatischen Rest bezeichnet, dadurch gekennzeichnet, daß man m- oder p-Methylthiobenzoylhalogenide der Formel

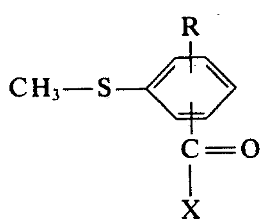

(II)

worin R und X die vorgenannte Bedeutung besitzen, mit Chlorgas in Abwesenheit von zusätzlichen Lösungsmitteln bei einer Temperatur von 25 bis 140°C umsetzt.

2. Verfahren nach Anspruch 1, dadurch gekennzeichnet, daß man die Reaktionstemperatur zu Beginn der Chlorierung bei 25 bis 45°C und dann nach kontinuierlicher Steigerung gegen Ende der Chlorierung bei 50 bis 140°C hält.

3. 3-Trichlormethylthiobenzoylchlorid.

4. 4-Chlor-3-trichlormethylthiobenzoylchlorid.

**Claims**

1. A process for the preparation of m- or p-trichloromethylthiobenzoyl halides of the formula

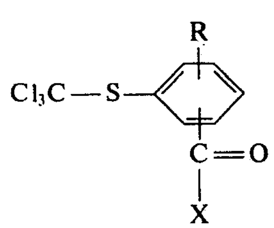

(I)

where X is halogen, and R is hydrogen, halogen or an aliphatic radical, wherein m- or p-methylthiobenzoyl halides of the formula

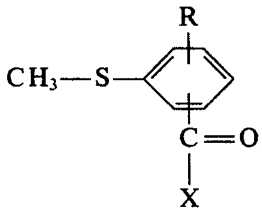

(II)

where R and X have the above meanings, are reacted with chlorine gas in the absence of additional solvents at a temperature of from 25 to 140°C.

2. A process as claimed in claim 1, wherein the reaction temperature is kept at 25 to 45°C at the beginning of the chlorination and then, after continuously rising, is kept at 50 to 140°C towards the end of the chlorination.

3. 3-Trichloromethylthiobenzoyl chloride.

4. 4-Chloro-3-trichloromethylthiobenzoyl chloride.

**Revendications**

1. Procédé pour la préparation d'halogénures de m- ou p-trichlorométhylthiobenzoyle de formule

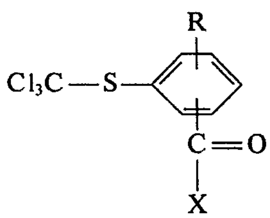

(I)

dans laquelle X représente un atome d'halogène, R est mis pour un atome d'hydrogène, un atome d'halogène ou un radical aliphatique, caractérisé en ce qu'on fait réagir des halogènures de m- ou p-méthylthiobenzoyle de formule

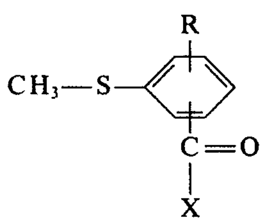

(II)

dans laquelle R et X ont les significations données ci-dessus, avec le gaz chloré en l'absence de solvants supplémentaires, à une température de 25 à 140°C.

2. Procédé selon la revendication 1, caractérisé en ce qu'on maintient la température de réaction à 25—45°C au début de la chloration, puis à 50—140°C vers la fin de la chloration, après une élévation continue.

3. Chlorure de 3-trichlorométhylthiobenzoyle.

4. Chlorure de 4-chloro-3-trichlorométhylthiobenzoyle.